# EUROPÄISCHE PATENTSCHRIFT

(11) **EP 2 136 766 B1**
(45) Veröffentlichungstag und Bekanntmachung des Hinweises auf die Patenterteilung: **15.01.2014**
(21) Anmeldenummer: 08716579.1
(22) Anmeldetag: 17.03.2008
(51) Int. Cl.: A61K 6/00, A61K 6/083

(54) **KONDITIONIERUNGSMITTEL UND VERFAHREN ZUM BINDEN VON HÄRTBAREN MISCHUNGEN**
CONDITIONING AGENT AND METHOD FOR BINDING HARDENABLE MIXTURES
AGENT DE CONDITIONNEMENT ET PROCEDE DE LIAISON DE MELANGES DURCISSABLES

(30) Priorität: 16.03.2007 DE 102007013285
(43) Veröffentlichungstag der Anmeldung: 30.12.2009
(73) Patentinhaber: S & C Polymer Silicon- und Composite-Spezialitäten GmbH, 25335 Elmshorn (DE)
(72) Erfinder: ENGELBRECHT, Jürgen, 22607 Hamburg (DE); GRÖGER, Gunther, 25335 Elmshorn (DE); GÖRLICH, Karl-Joachim, 64658 Fürth (DE)
(74) Vertreter: Forstmeyer, Dietmar
(86) Internationale Anmeldenummer: PCT/EP2008/002112
(87) Internationale Veröffentlichungsnummer: WO 2008/113541

(56) Entgegenhaltungen:
- EP-A1- 0 476 789
- EP-A1- 0 591 716
- GB-A- 2 341 393
- US-A- 5 354 827
- US-A- 6 031 016

## Beschreibung

Die Erfindung beschreibt ein Konditionierungsmittel, dessen Verwendung zur Vermittlung der Haftung von einer härtbaren Mischung an einem Formkörper und ein Verfahren zur Vorbehandlung der Oberflächen von Formkörpern aus gefüllten hochtemperaturbeständigen Kunststoffen zum Zwecke der besseren Haftung von härtbaren Mischungen auf diesen Formkörpern, sowie die hieraus resultierenden Verfahrensprodukte, insbesondere konditionierte Formkörper.

### Stand der Technik

Formteile aus hochtemperaturbeständigen Polymeren können in der Regel als weitgehend chemisch inert bezeichnet werden. Auch können sie in der Regel durch Lösungsmittel wie z.B. Aceton, Ethanol, Essigsäureethylester etc. nicht oder kaum angelöst werden.

Es ist oft erforderlich, an einen Formkörper aus hochtemperaturbeständigen Kunststoffen weiteres Material anzufügen. Dieses ist jedoch nicht ohne weiteres möglich, insbesondere wenn die Verbindung nicht nur optisch bestehen soll, sondern diese Verbindung auch mechanisch belastbar sein soll.

Als hochtemperaturbeständige Kunststoffe können z.B. Hochtemperatur-Thermoplaste genannt werden, die entweder amorph oder kristallin sein können, wie zum Beispiel Polyarylate, Polyarylensulfide, Polysulfone, flüssigkristallines Polymer (Liquid Crystal Polymer), insbesondere flüssigkristalline Polyester, Polyimide, Polyamidimide, Polyaryletherketone und Polyetheretherketone oder Polyoxymethylen.

Polymerisierbare Konditionierungsmittel auf Methacrylat-Basis zur Vorbehandlung der Oberflächen von anlösbaren, nicht hochtemperaturbeständigen Formkörpern aus Polyacrylat-, Polymethacrylat- und Polycarbonat-Kunststoffen zur Verbindung mit polymerisierbarem Methacrylat-Material sind bekannt.

EP 0 142 172 A2 beschreibt ein photopolymerisierbares Material auf Methacrylat-Basis, das sich als Klebstoff beziehungsweise Bindemittel zum Verbinden von Teilen aus Acrylat-Kunststoffen miteinander oder mit noch nicht gehärtetem Methacrylat-Material eignet. Es enthält neben einem Initiator für die Photopolymerisation als polare organische Verbindung Acrylsäure oder Methacrylsäure, vernetzend wirkende Dimethacrylate, Methacrylate oder Methylenchlorid als Verdünnungs- beziehungsweise Lösungsmittel. Es findet besondere Anwendung im Dentalbereich bei der Herstellung von Zahnprothesen aus Methacrylat-Material, um beschädigte Zahnprothesen zu reparieren bzw. die aus Acrylat-Kunststoff bestehenden künstlichen Zähne mit dem die Prothesenplatte bildenden Methacrylat-Material zu verbinden.

Ein photopolymerisierendes Klebmittel wird in DE 40 00 171 A1 in Anspruch genommen, das neben Methylmethacrylat, einem Methylmethacrylat-Polymer und einem Photoinitiator ein mehrfunktionelles Acrylat oder Methacrylat, vorzugsweise ein Tri- oder Tetraacrylat beziehungsweise -methacrylat, in einer Menge von 1 bis 20 Gewichtsprozent enthält. Dieses Klebmittel ist zum Verbinden von Formkörpern aus Acrylat-Kunststoff mit anderen Kunststoffformkörpern geeignet, wobei wenigstens einer der beteiligten Kunststoffformkörper für die zur Aushärtung verwendete Strahlung ausreichend durchlässig sein muss.

In EP 0 452 540 B1 wird ein Klebstoff zum Verbinden von Formteilen aus Polycarbonat-Kunststoffen miteinander erwähnt. Dieser Klebstoff enthält ein Peroxid/Amin-System als Katalysator für die Kaltpolymerisation und besteht aus zwei Komponenten, die vor Anwendung miteinander vermischt werden müssen. Neben dem Peroxid/Amin-System sind noch ein Alkylmethacrylat, 2,2-Bis-[4-(methacryloyloxyalkoxy)-phenyl]-propan, das Dimethacrylat eines Alkandiols- und/oder Trimethacrylat eines Alkantriols und ein Polymethylmethacrylat enthalten.

Aus EP 0 476 789 A1 ist ein orthodontisches Bracket bekannt, das aus Füllstoff-haltigem Methacrylatkunststoff besteht. Auf der an einem natürlichen Zahn mit Hilfe eines lichthärtenden Klebstoffs zu befestigenden Oberfläche trägt es eine dünne Schicht aus in dem Klebstoff teilweise löslichem oder quellbarem Methacrylatkunststoff. Die dünne Methacrylatkunststoff-Schicht wird durch Auftragen und Polymerisieren einer Mischung aus A) 30-70 Gewichtsprozent eines monofunktionellen Methacrylats, 30-70 Gewichtsprozent eines Methacrylat-Homo- oder -Copolymers und 0,01-1 Gewichtsprozent eines Polymerisationskatalysators oder B) 5-24 Gewichtsprozent eines mehrfunktionellen Methacrylats, 0-24 Gewichtsprozent eines monofunktionellen Methacrylats, 50-90 Gewichtsprozent eines Siliciumdioxid-Füllstoffs und 0,01-1 Gewichtsprozent eines Polymerisationskatalysators erhalten.

In EP 0 591 716 B1 wird ein polymerisierbares Konditioniermittel auf Methacrylat-Basis und ein Verfahren zur Vorbehandlung der Oberfläche von Formkörpern aus Polyacrylat-, Polymethacrylat- und Polycarbonat-Kunststoffen vor dem Auftragen von polymerisierbarem Methacrylat-Material und die Verwendung des Konditioniermittels beschrieben. Das Konditioniermittel oder der Klebstoff enthält 50-75 Gewichtsprozent Alkylmethacrylat oder ein Gemisch aus Alkylmethacrylat und Butandioldimethacrylat mit mindestens 50 Gewichtsprozent Alkylmethacrylat, 15-40 Gewichtsprozent Diurethandimethacrylat, 1-15 Gewichtsprozent Polymethylmethacrylat, 0-5 Gewichtsprozent Trimethacrylat, Tetramethacrylat oder ein Gemisch davon, 0,01-1 Gewichtsprozent monocyclischer Terpenkohlenwasserstoff und 0,1 Gewichtsprozent Polymerisationskatalysator. Das Alkylmethacrylat ist Methylmethacrylat und/oder Ethylmethacrylat, das Trimethacrylat ist Trimethylolpropantrimethacrylat und der monocyclische Terpenkohlenwasserstoff ist Terpinolen. Das Verfahren zur Vorbehandlung der Oberfläche von Formkörpern aus Polyacrylat-, Polymethacrylat- und Polycarbonat-Kunststoffen besteht darin, dass eine dünne Schicht des Klebstoffs oder Konditioniermittels aufgebracht wird.

Alle aufgeführte Druckschriften beschreiben ein Konditioniermittel bzw. einen Klebstoff zur Vorbehandlung der Oberflächen von Formkörpern aus nichthochtemperaturbeständigen, in der Regel anlösbaren Polyacrylat-, Polymethacrylat- und Polycarbonat-Kunststoffen zur Verbindung mit polymerisierbarem Acrylat- bzw. Methacrylat-Material.

### Aufgabe der Erfindung

Aufgabe der vorliegenden Erfindung war die Bereitstellung eines Konditioniermittels zur Vermittlung der Haftung von einer härtbaren Mischung an einem Formkörper aus hochtemperaturbeständigen Kunststoffen, das sinnfällige Herrichten des Konditionierungsmittels in der Form eines Kits, ein Verfahren zur Vorbehandlung der Oberflächen von Formkörpern aus gefüllten hochtemperaturbeständigen Kunststoffen zum Zwecke der besseren Haftung von härtbaren Mischungen auf diesen Formkörpern, sowie die hieraus resultierenden Verfahrensprodukte, insbesondere konditionierte Formkörper.

### Detaillierte Beschreibung der Erfindung

Erfindungsgemäß konnte die Aufgabe zumindestens für solche unanlösbaren, hochtemperaturbeständigen Kunststoffe gelöst werden, welchen Füllstoffe zugegeben sind.

Solche gefüllten Kunststoffe sind zum Beispiel beschrieben in WO 2006/108647.

Demgemäß konnte die gestellte Aufgabe gelöst werden durch ein:
Konditionierungsmittel zur Vermittlung der Haftung von einer härtbaren Mischung an einem Formkörper, der einen hochtemperaturbeständigen Kunststoff und einen Füllstoff umfasst, wobei das Konditionierungsmittel folgende Komponenten enthält:
   a) ein Haftmittel, welches an dem Füllstoff des Formkörpers und zumindest einer Komponente der härtbaren Mischung haften kann,
   b) gegebenenfalls einen oder mehrere Katalysatoren für die Vermittlung der Haftung der härtbaren Mischung am Füllstoff des Formkörpers und/oder für die Härtung der härtbaren Mischung,
   c) ein höhersiedendes Lösemittel mit Dipolcharakter,
   d) gegebenenfalls ein niedrigsiedendes Lösungsmittel,
   e) gegebenenfalls Zusätze wie Stabilisatoren, Inhibitoren, Antioxidantien und/oder Mono- bzw. Polyfunktionelle (Meth-) acrylate.

Um bei Anwendungen im Medizinbereich oder Dentalbereich Reizungen von lebendem Gewebe durch aus der Kunststoffmatrix hervorstehende Füllstoffpartikel zu minimieren, ist es von Vorteil, die hochtemperaturbeständigen gefüllten Thermoplaste mit härtbaren Mischungen abzudecken. Ein Abdecken der Formkörper mit einer härtbaren Mischung ist häufig aus ästhetischen Gründen erforderlich. Ferner kann hierdurch eine maßgeschneiderte geometrische Anpassung massengefertigter Formkörper an die individuellen Bedürfnisse der einzelnen Patienten erzielt werden. Diese Formkörper können im Dentalbereich z. B. Prothesen, Teile von Prothesen, Kronen, Brücken, Inlays, Onlays etc. sein. Auch die Kombination mit weichbleibenden Überzügen ist in diesem Zusammenhang von Vorteil.

Um die härtbaren Mischungen gut auf den Formkörpern haften zu lassen und die erfindungsgemäßen Konditionierungsmittel besonders gut zur Geltung kommen zu lassen, können die Formkörper in bevorzugter Weise durch mechanische Verfahren, wie z. B. Beschleifen, Sandstrahlen, Fräsen oder ähnliche Verfahren vorbehandelt sein.

Erfindungsgemäß wird das Konditioniermittel vorzugsweise zusammen mit dem Formkörper als Kit zur Verfügung gestellt, wobei optional zusätzlich die härtbare Mischung zur Verfügung gestellt wird. Demgemäß betrifft die vorliegende Erfindung auch ein Kit, umfassend
(i) einen Formkörper, der einen hochtemperaturbeständigen Kunststoff und einen Füllstoff umfasst,
(ii) ein Konditionierungsmittel, das ein Haftmittel und ein höhersiedendes Lösemittel mit Dipolcharakter umfasst, und (iii) gegebenenfalls eine härtbare Mischung.

Als geeignete gefüllte hochtemperaturbeständige Kunststoffe kommen in Frage Polyarylate, Polyarylensulfide, Polysulfone, flüssigkristallines Polymer, Polyimide, Polyetherimide, Polyamidimide, Polyaryletherketone oder Copolymerisate aus wenigstens zwei der vorstehend genannten Polymere oder ein Blend aus wenigstens zwei der vorstehend genannten Polymere. Besonders bevorzugt im Hinblick auf Anwendungen im Medizinbereich sind dabei Polyaryletherketone (PAEK) wie beispielsweise Polyetherketon (PEK), Polyetheretherketon (PEEK), Polyetherketonketon (PEKK), Polyetheretherketonketon (PEEKK) oder Polyetherketonetherketonketon (PEKEKK) wie sie in WO 2006/108647 A1 veröffentlicht sind.

In der vorliegenden Anmeldung sind die Begriffe "hochtemperaturbeständiger Kunststoff", "hochtemperaturbeständiges Polymer", "Hochtemperatur-Thermoplaste" und "hochtemperaturbeständige Thermoplaste" als Synonyme zu verstehen. Der Begriff "hochtemperaturbeständig" bedeutet, dass die Kunststoffe bis zu einer Temperatur von mindestens 200 °C, vorzugsweise bis zu einer Temperatur von mindestens 250 °C beständig sind, d.h. ihre Form nicht verändern. Diese mögliche starke Erwärmung der Formmasse bewirkt eine Verbesserung der mechanischen Eigenschaften sowie eine Verminderung von Eigenspannungen und Schwindungen sowie Verzug und führt dadurch zu einer besseren Dimensionsstabilität und Maßhaltigkeit bei verbesserten mechanischen Eigenschaften des Formteiles. Vor allem die mechanischen Eigenschaften werden in alle Richtungen stabilisiert, so dass ein isotropes Verhalten im Formteil entsteht, welches gleiche mechanische Eigenschaften in alle Richtungen aufweist. Dies ist insbesondere beim Einsatz im Dentalbereich wichtig, wo durch das Kauen und die Eigenbeweglichkeit der Zähne sehr starke torsische Belastungen in den Dentalformteilen auftreten können.

Die erfindungsgemäß eingesetzten Füllstoffe können anorganischer oder organischer Art sein wie mineralische Mehle oder Polymerisat-Pulver. Bevorzugt sind jedoch solche anorganischer Art, zum Beispiel solche mineralischen Mehle als Füllstoffe, wenn sie chemisch aktivierbar und verknüpfbar sind. Als Beispiele seien hier genannt: mikrofeine Glasfasern, Glaskugeln, Glasmehle, Kieselsäuren, Quarzmehl, Glimmer, Korund, Kaolin, Talkum, anorganische Pigmente, Apatite etc.

Die vorstehend genannten Füllstoffe können in Mengen von 1-90 Gewichtsprozent, bevorzugt 5-80 Gewichtsprozent, besonders bevorzugt 20-60 Gewichtsprozent, stärker bevorzugt 25-40 Gewichtsprozent, jeweils bezogen auf den Formkörper, enthalten sein. Sie können ihrerseits oberflächenbehandelt sein, zum Beispiel mit funktionellen Silanen wie z.B. Vinyl-, Methacryl-, Epoxy-, Amino-, Hydroxysilanen etc., Phosphorsäureestern, Phosphonsäureestern, Carbonsäureestern und/oder Mischungen davon.

Die auf die Formkörper aufzubringenden härtbaren Mischungen können verschiedener Art sei. Sie können polymerisierende Mischungen zum Beispiel auf Basis von Monomeren mit Vinyl-, Epoxid-, Isocyanat- oder anderen Gruppen sein. Bevorzugt sind polymerisierende Mischungen auf Basis von (Meth-)acrylaten, besonders bevorzugt solche, wie sie in dentalen Restaurationsmischungen üblich sind.

Das erfindungsgemäße Konditionierungsmittel enthält ein Haftmittel, welches mit den Füllstoffen des hochtemperaturbeständigen Kunstoffes und/oder mit Komponenten der härtbaren Mischung eine Bindung eingehen kann. Als Haftmitel können funktionelle Silane, also Silane mit funktionellen Gruppen, wie z.B. Vinyl-, (Meth-)acryl-, Epoxy-, Amino-, Hydroxysilane etc. und/oder Mischungen davon vorhanden sein. Weiterhin können funktionelle Phosphorsäure-, Phosphonsäure- und/oder Carbonsäureester und/oder Mischungen davon vorhanden sein. Das Haftmittel kann in dem Konditionierungsmittel in einer Menge von 0,1-100 Gewichtsprozent, bevorzugt 0,1- 80 Gewichtsprozent, stärker bevorzugt 1-50 Gewichtsprozent, noch stärker bevorzugt 1-25 Gewichtsprozent, jeweils bezogen auf das Konditionierungsmittel, vorhanden sein.

Gegebenenfalls kann das Konditionierungsmittel einen oder mehrere Katalysatoren für die Bindung der härtbaren Mischung am Füllstoff des hochtemperaturbeständigen Kunststoffes und/oder für die Härtung der härtbaren Mischung enthalten. Je nachdem von welcher chemischen Art die härtbare Mischung ist, kann das Konditionierungsmittel verschiedene Katalysatoren enthalten oder auch als Ein- und/oder Mehrkomponenten-System vorliegen. Mehrkomponenten-Systeme werden kurz vor dem Gebrauch miteinander vermischt.

Konditioniermittel können zum Beispiel Katalysatoren für Kalt-Heiß- und/oder Photopolymerisation enthalten. Zum Beispiel können diese für eine härtbare Mischung auf Basis von Methacrylaten und einer Kaltpolymerisation Peroxid/Amin-Systeme wie z.B. Dibenzoylperoxid/N,N-Dimethyl-p-toluidin und bei der Photopolymerisation Keton/Amin-Systeme wie z.B. Campherchinon/Amin, Acylphosphinoxid wie z.B. Diphenyl(2,4,6-trimethylbenzoyl)phosphinoxid sowie ein Propanon-Derivat und/oder Mischungen davon sein. Die Menge an Katalysator liegt bei vorzugsweise 0,1 bis 1,0 Gewichtsprozent, stärker bevorzugt 0,2 bis 0,7 Gewichtsprozent, jeweils bezogen auf das Konditionierungsmittel.

Erfindungsgemäß wichtig ist, daß das Konditionierungsmittel ein höhersiedendes Lösemittel mit Dipol-Charakter enthält. Vorzugsweise handelt es sich bei dem höhersiedenden Lösemittel mit Dipol-Charakter um ein aprotisches Lösemittel, das ein elektrisches Dipolmoment aufweist. Derartige höhersiedende Lösemittel weisen vorzugsweise einen Siedepunkt von mehr als 100 Grad Celsius bei Normaldruck, stärker bevorzugt von mehr als 110 Grad Celsius bei Normaldruck, noch stärker bevorzugt von mehr als 120 Grad Celsius bei Normaldruck auf. Auch wenn diese die hochtemperaturbeständigen Kunststoffe nicht richtig anlösen können, haben sie jedoch unerwartet eine positive Wirkung, wenn diese Kunststoffe gefüllt sind. Besonders geeignete höhersiedende Lösemittel mit Dipolcharakter sind z.B. Phenol, Diphenylsulfon, Cylohexanon, Acetylaceton und Ethylenglykol, besonders bevorzugt Dimethylsulfoxid. Die Menge an höhersiedendem Lösemittel mit Dipol-Charakter liegt bei 5 bis 99,9 Gew.-%, bevorzugt bei 10 bis 98 Gew.-%, stärker bevorzugt bei 20 bis 95 Gewichtsprozent, jeweils bezogen auf das Konditionierungsmittel.

Gegebenenfalls kann noch ein niedrigsiedendes Lösungsmittel Bestandteil des Konditionierungsmittels sein. Derartige niedrigsiedende Lösungsmittel weisen vorzugsweise einen Siedepunkt von bis zu 100 Grad Celsius bei Normaldruck auf. Diese Lösemittel können Wasser, Alkohole wie z.B. Methanol, Ethanol, Propanol etc, Ketone und/oder Diketone wie z.B. Aceton, Methylethylketon, Butandion etc. und/oder Mischungen davon, z.B. in Mengen von 0,1-90 Gewichtsprozent, bevorzugt 0,5-50 Gewichtsprozent, stärker bevorzugt 1-30 Gewichtsprozent, jeweils bezogen auf das Konditionierungsmittel, sein.

Neben den oben aufgeführten Komponenten können auch andere Zusätze nützlich sein. So können z.B. bei dem Aufbringen von härtbaren Mischungen auf Basis von Methacrylaten auf den Formkörper aus hochtemperaturbeständigen Kunststoff kleinere Mengen an Hydroxy(meth)acrylate wie z.B. Hydroxyethyl(meth)acrylat, Hydroxypropyl(meth)acrylat etc., Alkyl(meth)acrylate wie z.B. Methyl(meth)acrylat, Ethyl(meth)acrylat etc. und/oder Di- bzw. Poly(meth)acrylate wie z.B. Urethandi(meth)acrylate, Bis-phenol A di(meth)acrylate, Trimethylolpropandi(meth)acrylat, Triethylenglycoldi(meth)acrylat zugegeben werden.

Weitere Bestandteile können auch Stabilisatoren, Inhibitoren, Antioxidantien sein.

Es versteht sich von selbst, dass die obigen Ausführungen zu den einzelnen Komponenten, wie z.B. hochtemperaturbeständiger Kunststoff, Füllmittel, Haftmittel, höhersiedendes Lösemitteln etc. auch für die folgenden Ausführungsformen gelten.

Die vorliegende Erfindung umfasst ferner die Verwendung eines Konditionierungsmittels, umfassend ein Haftmittel und ein höhersiedendes Lösemittel mit Dipolcharakter, zur Vermittlung der Haftung von einer härtbaren Mischung an einem Formkörper, der einen hochtemperaturbeständigen Kunststoff und einen Füllstoff umfasst.

Die vorliegende Erfindung bezieht sich auch auf konditionierte Formkörper, umfassend einen hochtemperaturbeständigen Kunststoff und einen Füllstoff, wobei der Formkörper mit einem Konditionierungsmittel konditioniert ist und das Konditionierungsmittel ein Haftmittel und ein höhersiedendes Lösemittel mit Dipolcharakter umfasst. Der erfindungsgemäße konditionierte Formkörper kann zusätzlich mit einer härtbaren Mischung versehen sein.

Ferner bezieht sich die vorliegende Erfindung auf ein Verfahren zur Konditionierung von zumindest einem Teil der Oberfläche eines Formkörpers, der einen hochtemperaturbeständigen Kunststoff und einen Füllstoff umfasst, mit einem Konditionierungsmittel, das ein Haftmittel und ein höhersiedendes Lösemittel mit Dipolcharakter umfasst, wobei das Verfahren die folgenden Schritte umfasst:
- Auftragen des Konditionierungsmittels auf zumindest einen Teil der Oberfläche des Formkörpers,
- Einwirkenlassen des Konditionierungsmittels und
- optional Auftragen einer härtbaren Mischung auf die mit Konditionierungsmittel konditionierte Oberfläche des Formkörpers.

Der Ausdruck "zumindest einen Teil der Oberfläche des Formkörpers" bedeutet, dass mindestens 10 Flächen-% der Oberfläche des Formkörpers, vorzugsweise mindestens 50 Flächen-%, stärker bevorzugt mindestens 80 Flächen-%, am stärksten bevorzugt im wesentlichen vollständig, die Oberfläche des Formkörpers mit dem Konditionierungsmittel behandelt wird.

Die erfindungsgemäßen Konditionierungsmittel können bei Temperaturen bis 200 °C zur Anwendung kommen, vorzugsweise bis 150 °C, stärker bevorzugt bis 100 °C, besonders bevorzugt bei Raumtemperatur. Sie werden bevorzugt in einer dünnen Schicht von 0,01-0,2 mm aufgetragen und haben eine Einwirkzeit auf der Oberfläche des Formkörpers von 1-60 Minuten, vorzugsweise von 1-30 Minuten, besonders bevorzugt von 1-15 Minuten.

Die vorliegende Erfindung bezieht sich auch auf konditionierte Formkörper, die durch das obige Verfahren erhältlich sind.

Die erfindungsgemäßen konditionierten Formkörper können dentale Formkörper sein, bevorzugt Prothesen, Teilen von Prothesen, Kronen, Brücken, Inlays, Onlays etc..

Die erfindungsgemäßen, beschriebenen Konditionierungsmittel sind sehr gut geeignet, um eine gute Verbindung von härtbaren Mischungen zu hochtemperaturbeständigen Thermoplasten zu schaffen.

Anwendungen im Dentalbereich können zum Beispiel Verblendungen von vorgefertigten, wie z. B. gespritzten oder gefrästen Halbfertigkronen oder Stumpfkappen aus hochtemperaturbeständigen Thermoplasten, wie z. B. PEEK mit lichtpolymerisierbaren, ästhetischen Methacrylat-Composite-Massen als Verblendung sein.

Die Wirkung erfindungsgemäßer Konditionierungsmittel als Bindemittel zwischen lichthärtbarem Methacrylat-Composit (NEPA^{®}FIL, Fa. Merz Dental, Deutschland) und hochtemperaturbeständigem Glasfaser-gefülltem Thermoplast PEEK (Dentanium, Fa. Wegold, Deutschland) sollen folgende Beispiele näher erläutern.

Für die Haftwerte durch Scherung wurden Prüfkörper aus Dentanium mit Korund (50 *µ*m) gestrahlt, mit den verschiedenen Konditioniermitteln gemäß den Beispielen 1-6 benetzt und 2 Minuten einwirken gelassen. Danach wurde das Konditionierungsmittel mit einem sauberen Luftstrom verblasen und ein Zylinder mit einem Durchmesser von ca. 3 mm aus einem lichthärtbaren Dentalkunststoff (NEPA^{®}FIL, Fa. Merz Dental) aufgebracht und 90 Sekunden im Lichthärtegerät Dentacolor^{®} XS (Fa. Heraeus Kulzer) gehärtet.

Das Verfahren zur Messung der Scherung wurde vorzugsweise durchgeführt gemäß Göbel R und Welker D, Quintessenz Zahntech (2001) 27:197-203, Göbel R und Welker D, Quintessenz Zahntech (2000) 26:733-743, Göbel R und Welker D, ZWR (2004) 113:306-313.

Mit dem erfindungsgemäßen Ausführungsformen, insbesondere mit dem erfindungsgemäßen Verfahren, dem erfindungsgemäßen konditionierten Formkörper bzw. dem erfindungsgemäßen Konditionierungsmittel, können Messwerte gemäß dem obigen Messverfahren von mindestens 8 MPa, vorzugsweise mindestens 10 MPa, stärker bevorzugt mindestens 12 MPa, noch stärker bevorzugt mindestens 14 MPa erreicht werden.

Für den Hafttest durch Scherung lagerten die Prüfkörper 1 Stunde in Wasser von 60°C und wurden dann der Scherung unterworfen. Die Haftergebnisse sind im Folgenden zusammengefasst.

Beim Vergleichsbeispiel 1 wurde das Composite ohne weitere Konditionierung auf das Dentanium polymerisiert. (4,2 MPa).

Beim Vergleichsbeispiel 2 wurde das Composite mit nicht-erfindungsgemäßer Konditionierung mit Ethanol auf das Dentanium polymerisiert. (4,8 MPa).

Beim Vergleichsbeispiel 3 wurde das Composite mit nicht-erfindungsgemäßer Konditionierung mit DMSO auf das Dentanium polymerisiert. (5,2 MPa).

Beim Vergleichsbeispiel 4 wurde das Composite mit nicht-erfindungsgemäßer Konditionierung mit einem Methacrylsilan in Ethanol (Fantestic CerBond, R-Dental) auf das Dentanium polymerisiert. (5,0 MPa).

Beim erfindungsgemäßen Beispiel 5 wurde das Composite mit einem erfindungsgemäßen Konditionierungsmittel bestehend aus 98 Teilen DMSO und 2 Teilen Methacrysilan (Fantestic CerBond, R-Dental) auf das Dentanium polymerisiert. (15,2 MPa).

Beim erfindungsgemäßen Beispiel 6 wurde das Composite mit einem erfindungsgemäßen Konditionierungsmittel bestehend aus 50 Teilen DMSO, 48 Teilen Ethanol und 2 Teilen Methacrysilan (Fantestic CerBond, R-Dental) auf das Dentanium polymerisiert. (14,4 MPa).

## Patentansprüche

1. Verfahren zur Konditionierung der Oberfläche eines Formkörpers, der einen hochtemperaturbeständigen Kunststoff, ausgewählt aus Polyarylaten, Polyarylensulfiden, Polysulfonen, flüssigkristallinem Polymer, Polyimiden, Polyetherimiden, Polyamidimiden, Polyaryletherketonen, Copolymerisaten aus wenigstens zwei der vorstehend genannten Polymere oder einem Blend aus wenigstens zwei der vorstehend genannten Polymere, und einen Füllstoff umfasst, unter Verwendung eines Konditionierungsmittels, das ein Haftmittel und ein höhersiedendes Lösemittel mit Dipolcharakter, ausgewählt aus Dimethylsulfoxid, Phenol, Diphenylsulfon, Cylohexanon, Acetylaceton und Ethylenglykol, umfasst, wobei das Verfahren die folgenden Schritte umfasst:
- Auftragen des Konditionierungsmittels auf zumindest einen Teil der Oberfläche des Formkörpers,
- Einwirkenlassen des Konditionierungsmittels; und
- optional Auftragen einer härtbaren Mischung auf die mit Konditionierungsmittel konditionierte Oberfläche des Formkörpers.

2. Verfahren nach Anspruch 1, wobei das Konditionierungsmittel bei Temperaturen bis 200 °C, vorzugsweise bis 100 °C, stärker bevorzugt bei Raumtemperatur angewendet wird.

3. Verfahren nach Anspruch 1 oder 2, wobei die Einwirkzeit des Konditionierungsmittels auf der Oberfläche des Formkörpers 1-60 Minuten, vorzugsweise 1-30 Minuten, stärker bevorzugt 1-15 Minuten beträgt.

4. Konditionierter Formkörper, erhältlich nach einem Verfahren nach einem der Ansprüche 1 bis 3.

5. Konditionierter Formkörper nach Anspruch 4, **dadurch gekennzeichnet, dass** es sich um einen dentalen Formkörper handelt.

6. Konditionierter Formkörper nach Anspruch 5, **dadurch gekennzeichnet, dass** es sich um Prothesen, Teile von Prothesen, Kronen, Brücken, Inlays, Onlays etc. handelt.

7. Kit, umfassend
(i) einen Formkörper, der einen hochtemperaturbeständigen Kunststoff, ausgewählt aus Polyarylaten, Polyarylensulfiden, Polysulfonen, flüssigkristallinem Polymer, Polyimiden, Polyetherimiden, Polyamidimiden, Polyaryletherketonen, Copolymerisaten aus wenigstens zwei der vorstehend genannten Polymere oder einem Blend aus wenigstens zwei der vorstehend genannten Polymere, und einen Füllstoff umfasst,
(ii) ein Konditionierungsmittel, das ein Haftmittel und ein höhersiedendes Lösemittel mit Dipolcharakter, ausgewählt aus Dimethylsulfoxid, Phenol, Diphenylsulfon, Cylohexanon, Acetylaceton und Ethylenglykol, umfasst, und
(iii) gegebenenfalls eine härtbare Mischung.

8. Konditionierter Formkörper nach einem der Ansprüche 4 bis 6 oder Kit nach Anspruch 7, wobei der in dem Formkörper enthaltene Füllstoff Glasfasern, Glaskugeln, Glasmehl, Kieselsäuren, Quarzmehl, Glimmer, Korund, Kaolin, Talkum, anorganische Pigmente, Apatite und/oder Mischungen davon umfasst.

9. Konditionierter Formkörper nach einem der Ansprüche 4 bis 6, 8 oder Kit nach einem der Ansprüche 7 bis 8, wobei der Füllstoff z. B. mit funktionellen Silanen, Phosphorsäureestern, Phosphonsäureestern, Carbonsäureestern und/oder Mischungen davon oberflächenbehandelt ist.

10. Konditionierter Formkörper nach einem der Ansprüche 4 bis 6, 8 bis 9 oder Kit nach einem der Ansprüche 7 bis 9, wobei das Konditionierungsmittel ferner eine oder mehrere der folgenden Komponenten umfasst:
- einen oder mehrere Katalysatoren,
- ein niedrigsiedendes Lösemittel und/oder
- Zusätze, wie z.B. Stabilisatoren, Inhibitoren, Antioxidantien und/oder mono- bzw. polyfunktionelle (Meth-)acrylate.

11. Konditionierter Formkörper nach einem der Ansprüche 4 bis 6, 8 bis 10 oder Kit nach einem der Ansprüche 7 bis 10, wobei das Haftmittel ein funktionelles Silan, wie z. B. ein Vinyl-, (Meth-)acryl, Epoxy-, Amino-, Hydroxysilan und/oder eine Mischung davon ist oder ein funktioneller Phosphorsäure-, Phosphonsäure- und/oder Carbonsäureester und/oder eine Mischung davon ist.

12. Konditionierter Formkörper nach einem der Ansprüche 4 bis 6, 8 bis 11 oder Kit nach einem der Ansprüche 7 bis 11, wobei das Haftmittel Komponenten der härtbaren Mischung auf Basis von Monomeren mit (Meth-)acrylat-, Vinyl-, Epoxid-, Isoyanat- und/oder anderen Gruppen enthält.

13. Konditionierter Formkörper nach einem der Ansprüche 4 bis 6, 8 bis 12 oder Kit nach einem der Ansprüche 7 bis 12, wobei die härtbare Mischung auf Basis von Monomeren mit (Meth-)acrylat-, Vinyl-, Epoxid-, Isoyanat- und/oder anderen Gruppen aufgebaut ist.

14. Verwendung eines Konditionierungsmittels, umfassend ein Haftmittel und ein höhersiedendes Lösemittel mit Dipolcharakter, ausgewählt aus der Gruppe Dimethylsulfoxid, Phenol, Diphenylsulfon, Acetylaceton und/oder Ethylenglycol, zur Vermittlung der Haftung von einer härtbaren Mischung an einem Formkörper, der einen hochtemperaturbeständigen Kunststoff, ausgewählt aus Polyarylaten, Polyarylensulfiden, Polysulfonen, flüssigkristallinem Polymer, Polyimiden, Polyetherimiden, Polyamidimiden, Polyaryletherketonen, Copolymerisaten aus wenigstens zwei der vorstehend genannten Polymere oder einem Blend aus wenigstens zwei der vorstehend genannten Polymere, und einen Füllstoff umfasst.

## Claims

1. A method of conditioning the surface of a shaped article comprising a high-temperature-resistant plastic selected from polyarylates, polyarylene sulfides, polysulfones, liquid crystal polymer, polyimides, polyetherimides, polyamidimides, polyaryl ether ketones or copolymerisation products of at least two of the afore-mentioned polymers or a blend of at least two of the afore-mentioned polymers, and a filler, using a conditioning medium comprising an adhesive agent, and a higher-boiling solvent having a dipole character selected from dimethyl sulfoxide, phenol, diphenyl sulfone, cyclohexanone, acetylacetone and ethylene glycol, the method comprising the following steps:
- applying the conditioning medium to at least a part of the surface of the shaped article;
- allowing the conditioning medium to work in; and,
- optionally, applying a hardenable mixture to that surface of the shaped article which has been conditioned with conditioning medium.

2. The method according to claim 1, wherein the conditioning medium is used at temperatures up to 200°C, preferably up to 100°C, more preferably at room temperature.

3. The method according to claim 1 or 2, wherein the working-in time of the conditioning medium on the surface of the shaped article is 1-60 minutes, preferably 1-30 minutes, more preferably 1-15 minutes.

4. A conditioned shaped article obtainable by a method according to any one of the claims 1 to 3.

5. The conditioned shaped article according to claim 4, **characterised in that** it is in the form of a shaped dental article.

6. The conditioned shaped article according to claim 5, **characterised in that** it is in the form of dentures, parts of dentures, crowns, bridges, inlays, onlays etc.

7. A kit comprising
(i) a shaped article comprising a high-temperature-resistant plastic selected from polyarylates, polyarylene sulfides, polysulfones, liquid crystal polymer, polyimides, polyetherimides, polyamidimides, polyaryl ether ketones or copolymerisation products of at least two of the afore-mentioned polymers or a blend of at least two of the afore-mentioned polymers, and a filler,
(ii) a conditioning medium comprising an adhesive agent and a higher-boiling solvent having a dipole character selected from dimethyl sulfoxide, phenol, diphenyl sulfone, cyclohexanone, acetylacetone and ethylene glycol, and,
(iii) optionally, a hardenable mixture.

8. The conditioned shaped article according to any one of the claims 4 to 6 or the kit according to claim 7, wherein the filler present in the shaped article comprises glass fibres, glass spheres, glass flour, silicic acids, quartz flour, mica, corundum, kaolin, talc, inorganic pigments, apatites and/or mixtures thereof.

9. The conditioned shaped article according to any one of the claims 4 to 6, 8 or the kit according to any one of the claims 7 to 8, wherein the filler is surface-treated, for example, with functional silanes, phosphoric acid esters, phosphonic acid esters, carboxylic acid esters and/or mixtures thereof.

10. The conditioned shaped article according to any one of the claims 4 to 6, 8 to 9 or the kit according to any one of the claims 7 to 9, wherein the conditioning medium further comprises one or more of the following components:
- one or more catalysts,
- a low-boiling solvent and/or
- additives, for example stabilisers, inhibitors, antioxidants and/or mono- and/or poly-functional (meth)acrylates.

11. The conditioned shaped article according to any one of the claims 4 to 6, 8 to 10 or the kit according to any one of the claims 7 to 10, wherein the adhesive agent is a functional silane, for example a vinyl silane, (meth)acryl silane, epoxy silane, amino silane, hydroxy silane and/or a mixture thereof or is a functional phosphoric acid ester, phosphonic acid ester and/or carboxylic acid ester and/or a mixture thereof.

12. The conditioned shaped article according to any one of the claims 4 to 6, 8 to 11 or the kit according to any one of the claims 7 to 11, wherein the adhesive agent comprises components of the hardenable mixture based on monomers having (meth)acrylate, vinyl, epoxide, isocyanate and/or other groups.

13. The conditioned shaped article according to any one of the claims 4 to 6, 8 to 12 or the kit according to any one of the claims 7 to 12, wherein the hardenable mixture is formed on the basis of monomers having (meth)acrylate, vinyl, epoxide, isocyanate and/or other groups.

14. Use of a conditioning medium comprising an adhesive agent and a higher-boiling solvent having a dipole character selected from the group dimethyl sulfoxide, phenol, diphenyl sulfone, cyclohexanone, acetylacetone and ethylene glycol, in mediating in the adhesion of a hardenable mixture to a shaped article comprising a high-temperature-resistant plastic selected from polyarylates, polyarylene sulfides, polysulfones, liquid crystal polymer, polyimides, polyetherimides, polyamidimides, polyaryl ether ketones or copolymerisation products of at least two of the afore-mentioned polymers or a blend of at least two of the afore-mentioned polymers, and a filler.

## Revendications

1. Procédé servant au conditionnement de la surface d'un corps moulé, qui comprend une matière plastique résistante aux températures élevées, choisie parmi les polyarylates, les polyarylène sulfures, les polysulfones, le polymère cristallin liquide, les polyimides, les polyétherimides, les polyamidimides, les polyaryléther cétones, les copolymérisats composés d'au moins deux des polymères mentionnés ci-avant ou d'un mélange constitué d'au moins deux des polymères mentionnés ci-avant, et une charge, en utilisant un agent de conditionnement, qui comprend un adhésif et un dissolvant de type doublet présentant un point d'ébullition élevé, choisi parmi le diméthylsulfoxyde, le phénol, le diphénylsulfone, le cyclohexanone, l'acétylacétone et de l'éthylèneglycol, sachant que le procédé comprend les étapes suivantes consistant à :
- appliquer l'agent de conditionnement sur au moins une partie de la surface du corps moulé ;
- laisser agir l'agent de conditionnement ; et
- appliquer de manière optionnelle un mélange durcissable sur la surface du corps moulé conditionnée avec l'agent de conditionnement.

2. Procédé selon la revendication 1, sachant que l'agent de conditionnement est appliqué à des températures pouvant aller jusqu'à 200 °C, de préférence pouvant aller jusqu'à 100 °C, idéalement à température ambiante.

3. Procédé selon la revendication 1 ou 2, sachant que le temps d'action de l'agent de conditionnement sur la surface du corps moulé est compris entre 1 et 60 minutes, de préférence entre 1 et 30 minutes, idéalement entre 1 et 15 minutes.

4. Corps moulé conditionné, pouvant être obtenu selon un procédé selon l'une quelconque des revendications 1 à 3.

5. Corps moulé conditionné selon la revendication 4, **caractérisé en ce qu'**il s'agit d'un corps moulé dentaire.

6. Corps moulé conditionné selon la revendication 5, **caractérisé en ce qu'**il s'agit de prothèses, de parties de prothèse, de couronnes, de ponts, d'inlays, d'onlays, etc.

7. Kit comprenant
(i) un corps moulé, qui comprend une matière plastique résistante aux températures élevées, choisie parmi les polyarylates, les polyarylène sulfures, les polysulfones, le polymère cristallin liquide, les polyimides, les polyétherimides, les polyamidimides, les polyaryléther cétones, les copolymérisats composés d'au moins deux des polymères mentionnés ci-avant ou d'un mélange composé d'au moins deux des polymères mentionnés ci-avant, et une charge,
(ii) un agent de conditionnement, qui comprend un adhésif et un dissolvant de type doublet présentant un point d'ébullition élevé, choisi parmi le diméthylsulfoxyde, le phénol, le diphénylsulfone, le cyclohexanone, l'acétylacétone et de l'éthylèneglycol, et
(iii) éventuellement un mélange durcissable.

8. Corps moulé conditionné selon l'une quelconque des revendications 4 à 6, ou kit selon la revendication 7, sachant que la charge contenue dans le corps moulé est des fibres de verre, des billes de verre, du verre pulvérisé, des acides siliciques, de la poudre de quartz, du mica, du corindon, du kaolin, du talc, des pigments anorganiques, des apatites et/ou des mélanges de ces derniers.

9. Corps moulé conditionné selon l'une quelconque des revendications 4 à 6, 8 ou kit selon l'une quelconque des revendications 7 à 8, sachant que la charge comprenant par exemple des silanes fonctionnels, des éthers d'acide phosphorique, des éthers d'acide phosphonique, des éthers d'acide carboxylique et/ou des mélanges de ces derniers est traitée en surface.

10. Corps moulé conditionné selon l'une quelconque des revendications 4 à 6, 8 à 9 ou kit selon l'une quelconque des revendications 7 à 9, sachant que l'agent de conditionnement comprend en outre un ou plusieurs des composants qui suivent :
- un ou plusieurs catalyseurs ;
- un dissolvant présentant un point d'ébullition bas ; et/ou
- des additifs, tels que des stabilisateurs, des inhibiteurs, des antioxydants et/ou des (méth)acrylates mono-ou polyfonctionnels.

11. Corps moulé conditionné selon l'une quelconque des revendications 4 à 6, 8 à 10 ou kit selon l'une quelconque des revendications 7 à 10, sachant que l'adhésif est un silane fonctionnel, tel qu'un silane de vinyle, de (méth)acryl, un époxysilane, un aminosilane, un hydroxysilane et/ou un mélange de ces derniers ou un éther d'acide phosphorique fonctionnel, un éther d'acide phosphonique fonctionnel et/ou un éther d'acide carboxylique et/ou un mélange de ces derniers.

12. Corps moulé conditionné selon l'une quelconque des revendications 4 à 6, 8 à 11, ou kit selon l'une quelconque des revendications 7 à 11, sachant que l'adhésif contient des composants du mélange durcissable à base de monomères comprenant des groupes de (méth)acrylates, des groupes de vinyle, des groupes époxyde, des groupes isocyanate et/ou d'autres groupes.

13. Corps moulé conditionné selon l'une quelconque des revendications 4 à 6, 8 à 12 ou kit selon l'une quelconque des revendications 7 à 12, sachant que le mélange durcissable est établi sur la base de monomères comprenant des groupes de (méth)acrylates, des groupes de vinyle, des groupes époxyde, des groupes isocyanate et/ou d'autres groupes.

14. Utilisation d'un agent de conditionnement, comprenant un adhésif et un dissolvant de type doublet présentant un point d'ébullition élevé, choisi parmi le diméthylsulfoxyde, le phénol, le diphénylsulfone, l'acétylacétone et de l'éthylèneglycol aux fins de la promotion d'adhérence d'un mélange durcissable sur un corps moulé, qui comprend une matière plastique résistante aux températures élevées, choisie parmi les polyarylates, les polyarylène sulfures, les polysulfones, le polymère cristallin liquide, les polyimides, les polyétherimides, les polyamidimides, les polyaryléther cétones, les copolymérisats composés d'au moins deux des polymères mentionnés ci-avant ou d'un mélange composé d'au moins deux des polymères mentionnés ci-avant, et une charge.
